(19) Europäisches Patentamt
European Patent Office
Office européen des brevets

(11) Publication number: 0 039 091
B1

(12) **EUROPEAN PATENT SPECIFICATION**

(45) Date of publication of patent specification: 30.05.84

(51) Int. Cl.$^3$: C 07 D 307/94, A 61 K 31/34 // C07D307/32

(21) Application number: 81104399.1

(22) Date of filing: 01.05.80

(60) Publication number of the earlier application in accordance with Art. 76 EPC: 0 018 830

(54) **Spirobenzofuranone compounds.**

(30) Priority: 04.05.79 JP 55082/79
25.06.79 JP 80551/79

(43) Date of publication of application:
04.11.81 Bulletin 81/44

(45) Publication of the grant of the patent:
30.05.84 Bulletin 84/22

(84) Designated Contracting States:
BE CH DE FR GB IT LI NL SE

(56) References cited:
EP-A-0 003 084

**The file contains technical information submitted after the application was filed and not included in this specification**

(73) Proprietor: Takeda Chemical Industries, Ltd.
27, Doshomachi 2-chome Higashi-ku
Osaka-shi Osaka, 541 (JP)

(72) Inventor: Sugihara, Hirosada
5-604, 2 Minase 2-chome Shimamotocho
Mishima-gun Osaka 618 (JP)
Inventor: Watanabe, Masazumi
4-54, Seiwadainishi 2-chome Kawanishi
Hyogo 666-01 (JP)
Inventor: Kawada, Mitsuru
25-3, Tsukaguchicho 4-chome Amagasaki
Hyogo, 661 (JP)
Inventor: Imada, Isuke
18-10, Tsuruyamadai 4-chome Izumi
Osaka 594 (JP)

(74) Representative: Lewin, John Harvey
Elkington and Fife High Holborn House 52/54
High Holborn
London WC1V 6SH (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 039 091 B1

Courier Press, Leamington Spa, England.

**Description**

This invention relates to spirobenzofuranone compounds, a process for their preparation, and the use of the said compounds.

The present spiro compounds have the following formula (I):

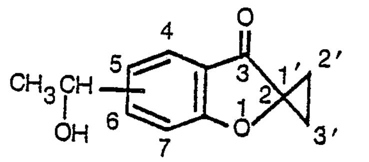

(I)

Referring to the above formula (I), the 1-hydroxyethyl group is preferably present at the 5- or 7-position.

The compound (I) of the present invention can be produced, for example, by subjecting a compound of the formula (III):

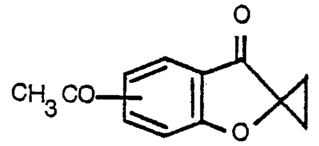

(II)

to reduction. For example, the reduction is carried out by treating a compound (II) with sodium borohydride in a suitable solvent under mild conditions which do not attack the 3-carbonyl group.

The compounds of this invention are of value as antiulcer, antiinflammatory and analgesic agents, and as drugs for the management of peptic ulcer, acute or chronic gastritis, lumbago, arthritis and other diseases. Management of a peptic ulcer in accordance with the present invention includes both the prophylactic administration of the spiro compounds (I) to prevent the outbreak of an ulcer in an ulcer prone patient, as well as the treatment of an existing peptic ulcer. In such medicinal applications, each compound (I) can be safely administered orally or parenterally, either as it is or as formulated with pharmaceutically acceptable carriers of diluents known *per se* into suitable dosage forms such as tablets, powders, capsules, injections and suppositories. While the recommended dosage depends on the subject, condition, route of administration, etc., the normal oral dosage for the treatment of peptic ulcer or acute or chronic gastritis is about 1 mg to 20 mg. as compound (I) per kg body weight per dose, to be given once to 3 times daily.

The starting compound (II) which is employed in the practice of this invention can be prepared by the procedure described in European Patent Publication No. 3084 published on July 25, 1979, or any process analogous thereto.

The following reference examples and working example are given to describe this invention in further detail.

Reference Example 1

α-bromo-γ-butyrolactone (12.5 g) was added to a mixture of methyl 3-acetylsalicylate (5.8 g) and potassium carbonate (10.4 g) in acetone (200 ml) under stirring, and the mixture was refluxed for 11 hours. The insolubles were filtered off and the filtrate was concentrated under reduced pressure. The residue was subjected to chromatography on silica gel eluting with chloroform. By the above procedure, there was obtained α-[(6-acetyl-2-methoxycarbonylphenyl)oxy]-γ-butyrolactone as a pale yellow oil.

Infra red adsorption spectrum (IR)$\gamma_{max}^{film}$ cm$^{-1}$:

1780 (γ-lactone), 1720 ($COOCH_3$), 1690 ($COCH_3$)

Elemental analysis, for $C_{14}H_{14}O_6$

Calcd.: C, 60.43; H, 5.07

Found: C, 60.21; H, 5.02

Reference Example 2

A mixture of α-[(6-acetyl-2-methoxycarbonylphenyl)oxy]-γ-butyrolactone (3.5 g), 1,8-diazabicyclo[5,4,0]-7-undecene (0.14 g) and sodium chloride (1.1 g) in N,N-dimethylformamide (66.5 ml) was stirred at 150—160°C for 5 hours. The solvent was distilled off under reduced pressure and the residue was subjected to chromatography on silica gel. The fraction eluted with dichloromethane was recrystallized from $CHCl_3$-hexane to give 7-acetylspiro [benzo[b]furan-2(3H), 1'-cyclopropane]-3-one (0.22 g) as colorless needles melting at 114—115°C.

Elemental analysis, for $C_{12}H_{10}O_3$

Calcd.: C, 71.28; H, 4.99

Found: C, 71.39; H, 4.96

Reference Example 3

A mixture of α-[(4-acetyl-2-methoxycarbonylphenyl)oxy]-γ-butyrolactone (2.8 g), 1,8-diazabicyclo[5,4,0]-7-undecene (0.056 g), sodium chloride (0.6 g) and N,N-dimethylformamide (28 ml) was heated at 150—155°C for 4 hours. The solvent was removed in vacuo and the resulting residue was dissolved in ethyl acetate. The ethyl acetate solution was washed with water and dried. The residue obtained by removal of the solvent in vacuo was chromatographed on silica gel. The fraction eluted with dichloromethane was crystallized from ethanol to give 5-acetyl-spiro[benzo[b]furan-2(3H), 1'-cyclopropane]-3-one (1.15 g) as colorless crystals.

IR$\gamma_{max}^{KRr}$ $cm^{-1}$ : 1700, 1680 ($CO,COCH_3$). NMR ($CDCl_3$)$\delta$ : 1.70 (4H, q. J = 7Hz, $CH_2$), 2.62 (3H, s, $COCH_3$), 7.20 (1H, d, J = 9Hz, aromat.H), 8.27, 8.30 (1H, dd, J = 9Hz, aromat.H), 8.29 (1H, d, J = 2Hz, aromat.H).

Example 1

$NaBH_4$ (0.9 g) was added portionwise to a well stirred solution of 5-acetylspiro[benzo[b]furan-2(3H), 1'-cyclopropane]-3-one (1 g) in tetrahydrofuran (25 ml) and isopropanol (3 ml) with cooling at −50°C. The reaction mixture was then stirred at room temperature for 30 minutes, followed by dilution with ice-water, which was neutralized with aqueous ammonium chloride. The aqueous solution was extracted with ethyl acetate. The extract was washed with water and dried. The residue obtained by removal of the solvent was chromatographed on silica gel, eluting with $CHC1_3$. The product was distilled under reduced pressure to give 5-(1-hydroxyethyl)spiro[benzo[b]furan-2(3H), 1'-cyclopropane]-3-one as a colorless oil.

b.p. 0.05 mmHz: 110°C (bath temperature). IR$\gamma_{max}^{film}$ $cm^{-1}$: 3350(OH), 1710 (CO). NMR ($CDCl_3$)$\delta$: 1.45 (3H, d, J = 6Hz, $CH_3$), 1.62 (4H, q, J = 3Hz, $CH_2$), 3.33 (1H, b, OH), 4.83 (1H, q, J = 6Hz, CH), 6.97 (1H, d, J = 9Hz, aromat.H), 7.55 (2H, m, aromat. H).

Elemental analysis, for $C_{12}H_{12}O_3$

Calcd.: C, 70.57; H, 5.92

Found: C, 70.47; H, 6.05

**Claims**

1. A spirobenzofuranone compound of the formula

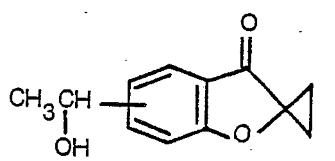

2. A compound according to Claim 1, which is 5(1-hydroxyethyl)spiro[benzo[b]furan-2(3H), 1'-cyclopropane]-3-one.

3. A compound according to Claim 1, which is 7-(1-hydroxyethyl)spiro[benzo[b]furan-2(3H), 1'-cyclopropane]-3-one.

4. A pharmaceutical composition which comprises, as an active ingredient, an effective amount of a compound as claimed in any of Claims 1 to 3, and a pharmaceutically acceptable carrier or diluent therefor.

5. A compound as claimed in any of Claims 1 to 3, or a pharmaceutical composition as claimed in Claim 4, for use in therapeutical treatment of a mammal.

6. A process for producing a spirobenzofuranone compound as claimed in any of Claims 1 to 3, characterized by subjecting a compound of the formula:

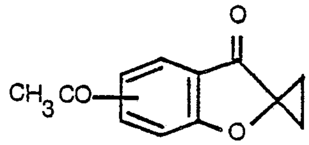

to reduction.

**Revendications**

1. Composé de spirobenzofuranone ayant la formule:

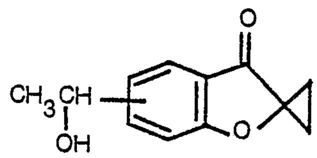

2. Composé selon la revendication 1, qui est la 5(1-hydroxyéthyl)spiro[benzo[b]furanne-2-(3H), 1′-cyclopropane]-3-one.

3. Composé selon la revendication 1, qui est la 7-(1-hydroxy-éthyl)spiro[benzo[b]furanne-2(3H), 1′-cyclopropane]-3-one.

4. Composition pharmaceutique qui comprend, comme ingrédient actif, une quantité efficace d'un composé tel que revendiqué dans l'une quelconque des revendications 1 à 3, et un véhicule ou un diluant pour ce composé, pharmaceutiquement acceptable.

5. Composé tel que revendiqué dans l'une quelconque des revendications 1 à 3, ou composition pharmaceutique telle que revendiquée dans la revendication 4, utilisable dans le traitement thérapeutique des mammifères.

6. Procédé de préparation d'un composé de spirobenzofuranone tel que revendiqué dans l'une quelconque des revendications 1 à 3, caractérisé par le fait qu'on soumet à la réduction un composé ayant la formule:

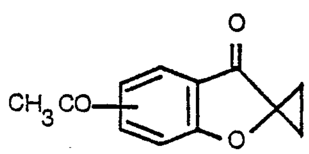

**Patentansprüche**

1. Spirobenzofuranon-Verbindung der Formel

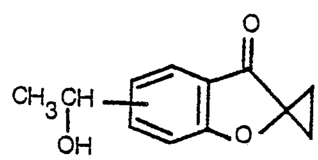

2. Verbindung nach Anspruch 1, die 5-(1-Hydroxyethyl)spiro[benzo[b]furan-2(3H),1′cyclopropan]-3-on ist.

3. Verbindung nach Anspruch 1, die 7-(1-Hydroxyethyl)spiro[benzo[b]furan-2(3H),1′cyclopropan]-3-on ist.

4. Pharmazeutische Zusammensetzung, enthaltend als Wirkstoff eine wirksame Menge der Verbindung nach irgendeinem der Ansprüche 1 bis 3 und ein pharmazeutisch unbedenkliches Trägermaterial oder Verdünnungsmittel für diese.

5. Verbindung nach irgendeinem der Ansprüche 1 bis 3 oder pharmazeutische Zusammensetzung nach Anspruch 4 zur Verwendung bei der therapeutischen Behandlung von Säugern.

6. Verfahren zur Herstellung einer Spirobenzofuranon-Verbindung nach irgendeinem der Ansprüche 1 bis 3, dadurch gekennzeichnet, daß eine Verbindung der Formel

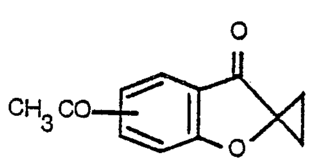

einer Reduktion unterworfen wird.